(19) Europäisches Patentamt
European Patent Office
Office européen des brevets

(11) Publication number: **0 421 577 A2**

# (12) EUROPEAN PATENT APPLICATION

(21) Application number: **90307543.0**

(51) Int. Cl.5: **B01J 13/20**, A61K 9/00

(22) Date of filing: **10.07.90**

(30) Priority: **10.07.89 US 377648**

(43) Date of publication of application:
**10.04.91 Bulletin 91/15**

(84) Designated Contracting States:
**AT BE CH DE DK ES FR GB GR IT LI LU NL SE**

(71) Applicant: **SYNTEX (U.S.A.) INC.**
**3401 Hillview Avenue**
**Palo Alto California 94304(US)**

(72) Inventor: **Lokensgard, David M.**
**1355 Daphne Drive**
**San Jose, California 95129(US)**

(74) Representative: **Armitage, Ian Michael et al**
**MEWBURN ELLIS 2 Cursitor Street**
**London EC4A 1BQ(GB)**

## (54) Solvent extraction process.

(57) There is disclosed a process for the extraction of volatile solvents entrained in a polymer-based pharmaceutical composition designed for sustained release of drug over an extended period of time prepared in microcapsule form wherein the composition comprises at least one hormonally active water-soluble polypeptide which process comprises the steps of contacting the composition with a stream of pressurized gas, and then removing the pressurized gas, and volatile solvents extracted from the pharmaceutical composition contained therein.

EP 0 421 577 A2

Xerox Copy Centre

## SOLVENT EXTRACTION PROCESS

This invention relates to an improvement in the process for producing a polymer-drug microcapsule pharmaceutical composition wherein the composition comprises a core containing at least one water soluble, hormonally active polypeptide and, optionally, a polymer hydrolysis modifying agent encapsulated in a bioderodable or biodegradable, biocompatible (co)polymer matrix. The improvement comprises a process for extracting volatile solvents entrained in the polymer-drug microcapsule pharmaceutical composition comprising the steps of (1) contacting the polymer-drug microcapsule pharmaceutical composition with a pressurized gas for a sufficient time to mobilize, and thereby extract from the microcapsules at least some of any residual solvents contained in the microcapsules and (2) then removing the pressurized gas, and the volatile solvents therein which have been extracted, from the polymer-drug microcapsule composition.

The use of supercritical fluids has been known and reported in the purification of polymers by extraction of residual monomer and catalyst and water (Ger. Pat. DE 3323940); the extraction of caffeine in the production of caffeine-free coffee extract (U.S. Pat. Publication 3,843,824); the purification of hop extract, produced by extracting a hop extract produced by extraction of hops with a liquid organic solvent and contaminated with solvent residue, by extracting with supercritical carbon dioxide (Ger. Pat. Publication DE 3,131,428); the extraction of an organic material from a solid by the solvent, namely supercritical $CO_2$, the pressure of the supercritical $CO_2$ being controlled with variation; and purifying maleic anhydride copolymer with olefins or vinyl aromatics by extraction with supercritical fluid to remove hazardous compounds (Ger. Pat. DE 300648).

The invention is an improvement in a process for producing a polymer-drug microcapsule pharmaceutical composition, wherein the drug is at least one hormonally-active water-soluble polypeptide in a therapeutically effective amount, wherein the improvement comprises the steps of

(1) contacting the microcapsules with a pressurized gas for a time sufficient to mobilize and thereby extract from the microcapsules at least some of any residual solvents contained in the microcapsules and then

(2) removing the pressurized gas and such residual solvents contained therein.

The terms used to describe this invention have their conventional physical/chemical meanings, that is, as follows: "gas" is a fluid that has neither independent shape or volume, in which molecules move freely, thereby causing the matter to expand indefinitely, occupying the total volume of any vessel in which it is contained; "fluid" is a substance tending to conform to the outline of its container; "liquid" is a non-gaseous fluid characterized by free movement of constituent molecules without the tendency to separate; "supercritical fluid" is a single-phase fluid above the critical pressure of that fluid at a given temperature; "pressurized gas" is a gas above ambient pressure for that temperature, but below critical pressure. A pressurized gas is also sometimes referred to as a "dense" gas.

This invention is an improvement in a process for producing a polymer drug microcapsule pharmaceutical composition, wherein the composition comprises at least one hormonally-active water-soluble polypeptide in a therapeutically effective amount encapsulated in a biocompatible, bioerodable, encapsulating polymer. Generally speaking, the process for producing a polymer-drug microcapsule pharmaceutical composition comprises the steps of providing the polymer matrix, dissolving the polymer matrix in a halogenated hydrocarbon solvent, dispersing the polypeptide in the polymer-solvent solution, adding an agent which is soluble in the halogenated hydrocarbon solvent but is a non-solvent for the polymer so as to cause the polymer to precipitate out of the halogenated hydrocarbon solvent onto the dispersed polypeptide droplets, thereby encapsulating the polypeptide. The microcapsules are then washed, hardened and then dryed. The improvement in this process comprises contacting the resulting microcapsules with a pressurized gas for a time and at a pressure sufficient to swell the polymer matrix to an extent sufficient to extract from the microcapsules residual volatile solvents contained in the microcapsules and removing the pressurized and volatile solvents contained therein.

For example, a poly(lactide-co-glycolide) polymer is dissolved in a halogenated hydrocarbon solvent, such as methylene chloride. An aqueous solution of polypeptide can is then added with rapid stirring to the solvent-polymer solution forming an emulsion. A second solvent-miscible material, which is a non-solvent for the polymer, such as silicone oil, is added with slow stirring to cause the polymeric excipient to precipitate out of the methylene chloride and collect on the water-solvent interface which coats the dispersed water droplets to give microcapsules.

After being formed, the microcapsules is washed, and hardened with a suitable organic solvent, (e.g., heptane) washed with water, washed with an aqueous non-ionic surfactant solution, and then dried at room

temperature under vacuum.

Although the pharmaceutical composition containing a hormonally-active water soluble polypeptide and the poly(lactide-co-glycolide) polymer in microcapsule form is suitable for its intended pharmaceutical use in treating human patients, the product tends to contain, or retain, some proportion of the solvents in the microcapsule product which are used in the manufacturing process. The residual methylene chloride and heptane, which are themselves volatile and readily evaporated in pure liquid form, appear to be entrapped, or entrained, within the spherical polymeric particles of the microcapsule product and thus rendered effectively non-volatile. While the concentration of the residual solvents in the microcapsule product is not believed to render the resulting pharmaceutical composition unsuitable or unsafe for human use, it is preferred to reduce the concentration of the residual solvents in the microcapsule product--in particular the halogenated hydrocarbon solvents such as dichloromethane (methylene chloride), and also, if possible other organic solvents, such as heptane--in the pharmaceutical composition. There is a need therefore for a process or method to eliminate or to substantially reduce the amounts of such residual solvents in the microcapsular pharmaceutical composition containing a LHRH analog in combination with a poly(lactide-co-glycolide) polymer while leaving the product otherwise intact.

One possible factor that may contribute to retention of these solvents within the microcapsules may be a very slow rate of diffusion of the solvents through the polymer matrix. The concentration of these solvents is not significantly reduced upon exposure to ambient pressure or reduced pressure (i.e. vacuum) for the ordinary time in the manufacturing process, e.g. several tens of hours to several days. The rate of diffusion of small molecules through a polymer can be increased by heating the material to temperatures above its glass transition temperature ($T_g$), or by expanding ("swelling") the polymer matrix by the introduction of solvents which dissolve in the solid material and thus increase its volume.

The physical form, chemical composition, and intended use of the PLGA-LHRH analog microcapsule product, however, limit the means available for removal of the solvents. For example, the product cannot be heated significantly in order to facilitate separation of the entrained solvents without softening the polymer, which results in individual particles adhering to each other (agglomeration). Agglomeration of the particles is undesirable since it interferes with the injectability of the microcapsules. In addition, heating might accelerate degradation of the drug contained within the microcapsules and might adversely affect the polymer itself.

Among the methods considered for investigations to remove the residual solvents from the polymer-drug, microcapsules was treatment of the microcapsules with supercritical $CO_2$ to extract the residual solvents. Carbon dioxide exists as a supercritical fluid at pressures above 1072 psi. (at 31 °C). However, such treatment resulted in varying degrees of agglomeration and fusion of the microspheres (microcapsules) to form a useless mass.

This invention is the unexpected and surprising discovery that a pressurized gas (sub-critical) is capable of swelling the polymer matrix of the polymer-drug microcapsule, such as a PLGA-LHRH analog, and can mobilize, for example, by dissolving, the entrained solvent residues, thereby allowing the solvent residues to diffuse to the surface of the particles and to evaporate. After contacting the microspheres with the pressurized gas, the pressurized gas can be removed, along with the volatile solvents by a conventional venting procedure, wherein residual solvents will be removed with the gas in the vapor phase.

The degree of swelling and efficiency of solvent extraction can be controlled by variation in the applied gas pressure. Desirably, the concentrations of halogenated hydrocarbon solvent (e.g. methylene chloride) will be reduced below detection limits. It is also possible but not critical that concentration of other organic solvents (e.g. heptane) may also be reduced; thus, the concentration of such other organic solvents need not be reduced in order for the process of this invention to be useful. Further, limits of gas pressure which allow the removal of residual solvents such as dichloromethane and heptane, without excessive swelling and consequent agglomeration have been determined.

Selection of conditions for swelling the polymer to increase the rate of diffusion of the entrapped solvents through the polymer is subject to several constraints. A pressurized gas selected for this purpose must interact strongly enough to penetrate the interior of each particle, and mobilize, for example, dissolve, the entrained solvent residues. A pressurized gas selected for the purpose of swelling the polymer must possess low toxicity since a residue of such solvent might be present after removal of the undesired processing solvents and drying of the swelled product. The degree of swelling must be controlled, since excessive swelling leads to softening of the material and can result in agglomeration or coalescence of the particles. The process must also leave the polypeptide, for example, an LHRH analog such as nafarelin acetate, intact and unextracted, and must not alter polypeptide distribution within the polymer matrix by, for example, dissolution of the individual polypeptide particles. Finally, the selected swelling agent must not react chemically with any component of the product and must not change the medically useful properties of

the product.

As the pressurized gas, there may be used $CO_2$ (carbon dioxide) and also mixtures of gases or liquids with pressurized gaseous $CO_2$ such as, for example, a gaseous or liquid pressurized C2-C8 alkane, such as $C_3H_8$ (n-propane), $C_5H_{12}$ (n-pentane) or $C_7H_{16}$ (n-heptane) in combination with pressurized gaseous $CO_2$. Other pressurized gases may be used which have, as described above, the properties of being able to swell the polymer, mobilize the entrained solvent residues, have low toxicity, and do not chemically alter the encapsulated polypeptide.

Depending on the pressurized gas selected, the limits of gas pressure which allow for removal of the residual solvents will vary, though they will not be greater than the supercritical pressure. For example, the lower pressure limit for effective mobilization of solvents by gaseous $CO_2$ or mixtures thereof with other gases, especially mixtures of $CO_2$ with $C_3H_8$ thereof at ambient temperature within the microcapsule product polymer (PLGA) matrix has been observed to be about about 110 psi of $CO_2$ pressure alone, while agglomeration of the individual particles has been observed to become significant at pressures above about 250 psi of $CO_2$ pressure alone. The addition of $C_3H_8$ has been found to extend the useful partial pressure of $CO_2$ upward. For example, especially preferred is the combination (or admixture) of pressurized gaseous $CO_2$ with liquid pressurized propane, e.g. propane, about 110 psi plus $CO_2$ to 400 psi. For the purposes of this invention, the pressure for $CO_2$ may be in the range of about 100 - 300 psi, preferrably 110 - 250 psi; and when in the presence of a gaseous or liquid pressurized alkane, the pressure range may be about 100 - 500 psi, preferably about 110 - 400 psi.

The polypeptide used in the composition subjected to the process according to this invention is preferably an LHRH-active polypeptide, including LHRH itself and synthetic analogs thereof and pharmaceutically acceptable salts thereof, which act on the anterior pituitary gland to effect the release of hormones which affect the activity of reproductive organs. For purposes of this application, the expression "LHRH analog" is meant to encompass LHRH itself as well as the synthetic analogs thereof and pharmaceutically acceptable salts thereof.

LHRH analogs include compounds having agonist or antagonist effects. Representative LHRH agonists include, but are not limited to, those compounds that are disclosed in Nestor et al., U.S. Patent 4,234,571. Representative LHRH antagonists include, but are not limited to, those compounds disclosed in Nestor et al., U.S. Patent 4,801,577.

Other representative LHRH analogs include those nona- and decapeptides having LHRH agonist or antagonist activity disclosed, along with processes for preparation thereof, in the following U.S. Patents No.: 3,813,382; 3,843,065; 3,849,389; 3,855,199; 3,886,135; 3,890,437; 3,892,723; 3,896,104; 3,901,872; 3,914,412; 3,915,947; 3,929,759; 3,937,695; 3,953,416; 3,974,135; 4,010,125; 4,018,914; 4,022,759; 4,022,760; 4,022,761; 4,024,248; 4,034,082; 4,072,668; 4,075,189; 4,075,192; 4,086,219; 4,101,538; 4,124,577; 4,124,578; 4,143,133; 4,253,997; 4,292,313; and 4,341,767.

The LHRH agonist compounds of greatest interest, and thus more preferred, herein are those polypeptide compounds that are the subject of US Patent 4,234,571. including their pharmaceutically acceptable salts. The disclosure of this patent is incorporated herein by reference. These polypeptides are nonapeptides and decapeptides represented by the formula:

(pyro)Glu-His-V-Ser-W-X-Y-Arg-Pro-Z (I)

and the pharmaceutically acceptable salts thereof wherein

V is Trp (tryptophyl), Phe (phenylalanyl), or Nal (3-(1-naphthyl)-L-alanyl)

W is Tyr (tyrosyl), Phe, or 3-(1-pentafluorophenyl)-L-alanyl;

X is a D-amino acid residue

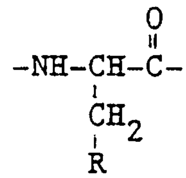

wherein R is

(a) a carbocyclic aryl-containing radical selected group consisting of naphthyl, anthryl, fluorenyl, phenanthryl, biphenyl, benzhydryl and phenyl substituted with three or more straight chain lower alkyl groups; or

(b) a saturated carbocyclic radical selected from the group consisting of cyclohexyl substituted with three or more straight chain lower alkyl groups, perhydronapthyl, perhydro-2,2-diphenylmethyl and adamantyl;

Y is leucyl, isoleucyl, nor-leucyl or N-methyl-leucyl; and

Z is glycinamide or -NH-$R^1$ wherein

$R_1$ is lower alkyl, cycloalkyl, fluoro lower alkyl or

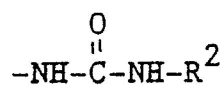 ; and

$R_2$ is hydrogen or lower alkyl.

Most preferred LH-RH agonists are those having formula (I) above wherein X is 3-(2-naphenylalanyl-D-alanyl or 3-(2,4,6-trimethylphenyl)- D-alanyl; Z is glycinamine; V is tryptophyl or phenylalanyl; W is tyrosyl and Y is leucyl or N-methylleucyl.

Especially preferred among the LHRH agonists, for use in the process according to this invention, is a polypeptide represented by the formula:

(pyro)Glu-His-Trp-Ser-Tyr-3-(2-napththyl)-D-Ala-Leu-Arg-Pro-Gly-$NH_2$ (nafarelin acetate).

The LHRH antagonist compounds of greatest interest, and thus, more preferred, herein are those polypeptide compounds that are the subject of U.S. Patent No. 4,801,577. These polypeptides are nonapeptides and decapeptides represented by the formula:

A-B-C-Ser-D-E-F-G-Pro-J (II)

or a pharmaceutically acceptable salt thereof, wherein:

A is an amino acyl residue selected from the group consisting of either the D- or the L- isomer of: N-Ac-D,L-$\Delta^{3,4}$-prolyl, N-Ac-D,L-prolyl, N-Ac-D,L-phenylalanyl, N-Ac-D,L-p-chlorophenylalanyl, N-Ac-D,L,-p-fluorophenylalanyl, N-Ac-3-(1-naphthyl)-D,L-alanyl, N-Ac-3-(2-naphthyl)-D,L-alanyl, and N-Ac-3-(2,4,6-trimethylphenyl)-D,L-alanyl;

B is an amino acyl residue selected from the group consisting of D-phenylalanyl, D-p-chlorophenylalanyl, D-p-fluorophenylalanyl, D-p-nitrophenylalanyl, 2,2-diphenylglycyl, D-$\alpha$-methyl-p-chlorophenylalanyl and 3-(2-naphthyl)-D-alanyl;

C is an amino acyl residue selected from the group consisting of D-tryptophanyl, D-phenylalanyl, 3-(3-pyridyl)-D-alanyl, and 3-(2-naphthyl)-D-alanyl;

D is an amino acyl residue selected from the group consisting of L-phenylalanyl, L-tyrosyl, and 3-(3-pyridyl)-alanyl, arginyl, or G;

E is 3-(2-naphthyl)-D-alanyl, 3-(3-pyridyl)-D-alanyl, D-tyrosyl, D-tryptophanyl, D-nicotinyl-lysyl, pyridylacetyl-lysyl, D-Glu(AA) or G;

F is an amino acyl residue selected from the group consisting of L-leucyl, L-norleucyl, L-phenylalanyl, L-tryptophanyl, and 3-(2-naphthyl)-L-alanyl;

G is an amino acyl residue selected from the group consisting of the radicals represented by the following structural formulas:

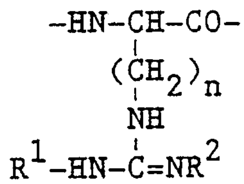

wherein

n is 1 to 5;

$R^1$ is alkyl of 1 to 6 carbon atoms or fluoroalkyl;

$R^2$ is hydrogen or $R^1$; or $R^1$-HN-C=$NR^2$ is a ring represented by the following structural formulas:

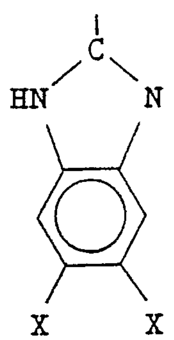
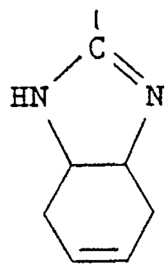
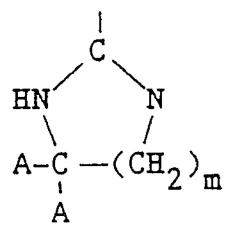

wherein m is 1 to 4; A is hydrogen or alkyl of 1 to 6 carbon atoms; and X is halo or A; and

(b)

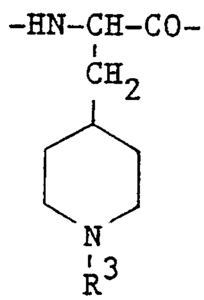
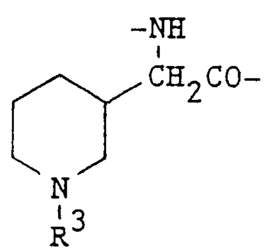

wherein $R^3$ is hydrogen, alkyl of 1 to 6 carbon atoms, phenyl or phenyl loweralkyl; and

J is D-alaninamide; D-leucinamide; glycinamide; or $-NHR^4$ wherein $R^4$ is lower alkyl or $NHCONH_2$.

Still more preferred LHRH antagonists are those wherein A is N-Ac-D-Nal(2) or N-Ac-D-pCl-Phe; B is D-pF-Phe or D-pCl-Phe; C is D-Trp, D-Nal(2) or Pal(3); D is Pal(3), Tyr, Arg, Deh, Mbh, Bth, or Pha; E is D-Trp, D-Tyr, D-Nal(2), D-Pal(3), D-Deh, D-Mbh, D-Pha or D-Bth; F is Leu or Phe; G is Deh, Bth, Mbh, or Pha; and J is D-$AlaNH_2$ or $GlyNH_2$.

Most preferred LHRH antagonists are those wherein:

- A is N-Ac-D-Nal(2);
- B is D-pCl-Phe;
- C is D-Trp or D-Pal(3);
- D is Tyr, Arg, Deh, Mbh, Bth or Pha;
- E is D-Trp, D-Pal(3), D-Nal(2), D-Tyr, D-Deh, -Mbh, D-Bth or D-Pha;
- F is Leu;
- G is Deh, Mbh, Bth or Pha; and
- J is D-$AlaNH_2$.

Especially preferred among said LHRH antagonist are polypeptides represented by the formula:

A-B-C-Ser-D-E-F-G-Pro-J (II)

wherein A in N-Ac-D-Nal(2), B is D-pCl-Phe, C is D-Pal(3), D is Tyr, E is selected from D-Deh and D-Pal(3), F is Leu, G is Deh, and J is Ala-$NH_2$. Most preferably when the polypeptide of Formula (II), wherein A, B, C, D, E, G, and J have all of the above meanings, E is D-Deh.

Kent et al., U.S. Patent 4,675,189 discloses novel sustained release microcapsule compositions comprising water-soluble, hormonally-active polypeptides and, optionally, a polymer hydrolysis modifying agent, encapsulated in a biocompatible, biodegradable polymer.

More particularly, the patent covers a pharmaceutical composition designed for sustained release over a period of at least 1 month of a luteinizing hormone-releasing hormone (LHRH) analog, prepared in microcapsule form, comprising at least one LHRH analog or pharmaceutically acceptable salt thereof in an amount between about 0.01 and 40.0 weight %, of the polymer used for encapsulation, preferrably between 0.1 and 10.0 weight %.

A number of polymers have been developed which meet the criteria of being biocompatible and biodegradable and/or biodegradable. Examples of suitable polymers are also disclosed in Kent et al., U.S. Patent No. 4,675,189.

Preferably, the polymer matrix used in the composition subjected to the process according to this invention is a polymer prepared from lactic acid as the sole monomer or as the principal monomer and glycolic acid as the comonomer, the latter copolymer being referred to as "poly(lactide-co-glycolide) copolymers" or "poly(lactic-co-glycolic acid polymers" (PLGA).

The combinations of preferred monomer and comonomer which can be prepared are numerous but the most effective polymer matrices are those polymers prepared from lactic acid alone or lactic acid and glycolic acid wherein the glycolic acid is present as a comonomer in a molar ratio of 100:0 to about 40:60 (lactic:glycolic). Most preferably there is used a poly(lactic-co-glycolic acid) coplymer (PLGA) having a molar ratio between 75:25 and 50:50.

Poly(lactic-co-glycolic acid) polymers preferably will range in molecular weight from about 20,000 to about 100,000. The molecular weight of a particular polymer is independent of its monomeric makeup. Thus, polymers can be varied both as to their monomer composition as well as to their molecular weight and be within the scope and intent of the polymer used in the composition subjected to the process according to this invention.

The preparation of PLGA polymer matrices and of polymer-drug microcapsules is disclosed in Kent et al., US Patent No. 4,675,189 and Boswell et al., US Patent 3,773,919, the disclosure of which is incorporated herein by reference.

Halogenated organic solvents which may be used in the preparation of the PLGA polymer matrices are the $C_1$ to $c_4$ halogenated hydrocarbons such as, for example, methylene chloride, ethylene dichloride, ethylene chloride, 2,2,2-trichloroethane and the like.

The non-solvent, called a coacervation agent may be any solvent miscible polymeric, mineral oil or vegetable oil compounds which are non-solvents for the encapsulating polymers. There may be used, for example, silicone oil, peanut oil, soybean oil, corn oil, cotton seed oil, coconut oil, linseed oil, and the like.

After being formed, the microcapsules may be subjected to one or all of the following additional steps: washing, hardening and drying. For example they may be washed and hardened with a suitable organic solvent, washed with water, washed with an aqueous non-ionic surfactant solution, and then dried at room temperature under reduced pressure (vacuum).

Microcapsules may range in diameter from about 1 to about 500 microns, depending upon the techniques employed. Preferably, the microcapsule diameter will be between about 5 and 200 microns.

The following examples, including tables and figures herein described, are illustrative of but a few embodiments of the invention and are not to be construed as limiting in scope. All parts and percentages are by weight and all temperatures are in degrees Celsius unless otherwise indicated.

## COMPARATIVE EXAMPLE 1

This example illustrates the unsuccessful efforts to further purify PLGA-nafarelin acetate microcapsules by employing conventional supercritical $CO_2$ extraction technology. The general technique is described in of "Dense Gases for Extraction and Refining" Sthal, Quirin and Gerard, 1986, Springer-Verlag, Berlin, section: III.2C Preparative extraction, Pages 44-46.

Three experiments were performed in which supercritical $CO_2$ was contacted with samples of PLGA

microcapsules.

B.1 A sample of 3.8 g of PLGA microcapsules was loaded into a conventional tubular extractor. Employing conventional techniques, carbon dioxide at 25-27 degrees C., and 4000 psi was pumped through the tubular extractor containing the sample at 4-5 g/min for 2.0 hours. The sample fused into a solid pellet.

B.2 A sample of 0.3 g of PLGA microcapsules was loaded into a glass high pressure reaction flask having a gas/liquid inlet and the flask was sealed. Carbon dioxide (1000 psi) was slowly introduced into the flask to maintain the temperature below ambient temperature. The microcapsule sample immediately agglomerated when contacted with the carbon dioxide.

B.3 This experiment is a modification of Example B.2 above. A sample of 0.3 g of PLGA-nafarelin acetate microcapsules was loaded into a glass high pressure reaction flask having a gas/liquid inlet and the flask was sealed. The flask was filled with liquid carbon dioxide at 1000 psi. The flask was held at room temperature and was occasionally shaken by hand for a period of 1 hour. Then, the carbon dioxide was drained. This procedure was repeated twice. The microcapsule sample agglomerated under these experimental conditions.

Although the application of conventional supercritical $CO_2$ extraction technology effected agglomeration and was thus unsatifactory because such treatment destroyed product integrity, analysis (conventional gas chromatography) of the residues showed that methylene chloride concentration in the resulting product mass was below detectable limits and that alkane concentration ($C_7H_{16}$ and $C_5H_{12}$) was less than 0.5%. Typical control (non-extracted) concentrations of methylene chloride and heptane in the PLGA-nafarelin acetate microcapsules are between 0.5 to 2.5% and 8 to 12%, respectively, and in the above instance, are 0.16% and 11.3%, respectively.

EXAMPLE 1

A sample of the product (PLGA-nafarelin acetate microcapsules according to Example A) was placed within a steel pressure vessel and, by means of appropriate control apparatus, was exposed to a slowly flowing stream of carbon dioxide gas at pressures which varied smoothly and in a cyclic fashion from vacuum (1 mm Hg) to a higher pressure (about 250 psi). Reduction of pressure from the maximum value was carried out slowly in order to prevent disruption of the product particles as a result of internal pressure. After sixty-five repetitions of the pressure cycle, the sample was removed and assayed for its solvent content and for its performance in an in vitro nafarelin acetate release test, which predicts the pharmaceutical performance of the material.

The solvent content of the unprocessed sample was 6.0% heptane and 400 ppm (parts per million) (0.04%) dichloromethane, while the sample subjected to gas extraction contained 5.1% heptane and less than 80 ppm (0.008%) dichloromethane. 80 ppm is the detection limit of the analysis procedure for dichloromethane. The in vitro drug release assay showed that the processing of the sample had not affected its pharmaceutical performance characteristics, and microscopic examination of the product before and after extraction showed no significant changes in particle shape or size.

EXAMPLE 2

A second sample of the product was extracted in a similar fashion to that described in Example 1, except that the gas pressure was varied between limits of 160 and 230 psi. This sample initially contained 9.9% heptane and 0.66% (6600 ppm) dichloromethane. After the treatment with pressurized carbon dioxide, the heptane content was reduced to 8.6%, and the dichloromethane level was less than 80 ppm, demonstrating that the application of vacuum during the process is not necessary.

EXAMPLE 3

This example illustrates the effect of the use of pressurized hydrocarbon(s) in combination with pressurized gaseous $CO_2$ wherein the liquid pressurized $CO_2$ is used to swell the polymer matrix. The samples were contacted with the pressurized gas set forth in each experiment employing conventional technology.

The "sight-cell" used in the following experiment was a Transparent Liquid Level Gage #1 available

from Inferno Manufacturing Support Corporation, Shreve Port, Louisiana. The gauge was filled at each end with valves, a pressure gauge, fill lines, etc. It was loaded with samples by unscrewing the fittings from one end, filling with sample and replacing the fitting. The use of cotton plugs in each end kept the sample suspended in liquified gases in the visible portion. Internal working volume was 50 ml. The solvent residues are measured as w/w% (weight of solvent/weight of microspheres). The control (untreated) concentrations of methylene chloride and heptane in this example are about 0.16% and 11.3%, respectively.

3.1 A sample of 0.4 g of PLGA microcapsules was loaded in a glass and stainless steel sight cell. About 50 ml of liquid propane (LP) was introduced (75% full sight cell) at RT and at its vapor pressure of 110 psig. The cell was shaken occasionally over a 1 hr period, then allowed to stand overnight. The LP was drained out in morning and the cell was recharged with fresh LP; The cell was shaken occasionally during the next 8 hr period. The cell was again allowed to stand overnight, and the draining, filling and shaking procedure was repeated once more. The total elapsed time of the PLGA microcapsules in LP was 42 hr. The microcapsules became a free-flowing slurry when suspended in LP. After depressurizing and unloading the cell, the powder appeared same as before, perhaps less sticky and more free-flowing. About 0.05% of $CH_2Cl_2$ and 9% of heptane was detected.

3.2 A sample of about 0.8 g of PLGA microcapsules was loaded into a sight cell. About 50 ml of LP was introduced and $CO_2$ added stepwise. The cell was shaken after each carbon dioxide addition to observe stickiness of supended microcapsules. Steps correspond to total cell pressures of: 110 (LP only); and with added $CO_2$: 160, 180, 200, 250, 300, 400 psi. The microcapsules became progressively stickier until it was all adhering in clumps to inside cell surfaces at 400 psi. This was allowed to stand overnight in mixed liquid phase (19.5 hrs.), drained and depressurized. The microcapsules lightly fused into pliable, open-structured lumps. No $CH_2Cl_2$ was detected and 1.0% of heptane was detected.

3.3 A sample of 1.1 g of PLGA microcapsules was loosely packed in a transparent 1.0 cm ID plastic tube mounted inside the sight cell. Carbon dioxide at 18-20 C and 200 psi (constant) was passed through the tube for 24 hr at a rate of 20-30 ml/min (STP). The column of microcapsules (powder) had solidified but was easily reground in the mortar and pestle to its original consistency. No residual $CH_2Cl_2$ was detected and 5.8% of heptane was detected.

3.4 A sample of 0.5 g of PLGA microcapsules was ground to a fine powder and loaded into a sight cell with 25 ml of heptane and the cell was sealed. Carbon dioxide at 18-20 °C was slowly introduced into the cell until total pressure was 200 psi. The cell was shaken occasionally, then allowed to stand overnight and finally the heptane and carbon dioxide was drained. The total time under carbon dioxide pressure was about 18 hr. Clumped powder samples were removed. No $CH_2Cl_2$ was detected and 4.3% of heptane was detected.

3.5 The procedure described in example 4.4 above was repeated except that 25 ml of pentane was substituted for heptane and the carbon dioxide pressure was 400 psi and the total time under carbon dioxide pressure was about 4 hrs. Clumped powder samples were removed. About 0.125% of $CH_2Cl_2$ and 0.85% of heptane was detected. Thus, insufficient $CH_2Cl_2$ was removed.

3.6 A sample of 4.28 g of finely powdered PLGA microcapsules was placed in a 60 ml fritted glass filter funnel (medium porosity 4.0 cm diameter) to a depth of 0.6 cm. The funnel was loaded upright in a conventional pressure vessel. The funnel stem was attached to a carbon dioxide inlet tube. The vessel was sealed and then pressurized with carbon dioxide to 200 psi and carbon dioxide began flowing upward through the funnel. The vessel was maintained at 200 psi of carbon dioxide introduced at a flow rate of 0.3 cfm (STP) (measured with a rotameter) for 24 hrs at a temperature of 19-20 °C. Then the vessel was vented and the sample was removed as a solid disc. No $CH_2Cl_2$ was detected but about 7.4% of heptate was detected.

The results of Examples B.1, B.2, 3.1, 3.2, 3.3, 3.5 and 3.6 are summarized in Table I.

TABLE I

| EX. NO. | PRESSURIZED GAS(ES) | $MeCl_2$ w/w% | HEPTANE w/w% | COMMENTS |
|---|---|---|---|---|
| B.1 | $CO_2$, 4,000 psi | | | Destroyed microspheres |
| B.2 | Liquid $CO_2$ (1,000 psi) | | | Destroyed microspheres |
| 3.1 | Propane, 100 psi | 0.05 | 9 | No extraction |
| 3.2 | Propane, 110 psi; added $CO_2$ to 400 psi | N.D.* | 1.0 | |
| 3.3 | $CO_2$, 200 psig | N.D.* | 5.8 | Microspheres arranged in a packaged tubular column |
| 3.4 | $CO_2$ at 200 psig plus heptane | N.D.* | 4.3 | |
| 3.5 | $CO_2$ at 200 psi; plus pentane to 400 psi | 0.125 | 0.85 | |
| 3.6 | $CO_2$, 200 psig | N.D.* | 7.4 | Microspheres arranged in a thin bed |

* Not detectable

## Claims

1. An improvement in a process for producing a polymer-drug microcapsule pharmaceutical composition, wherein the drug is at least one hormonally-active water-soluble polypeptide in a therapeutically effective amount, wherein the improvement comprises the steps of
   (1) contacting the microcapsules with a pressurized gas for a time sufficient to mobilize and thereby extract from the microcapsules at least some of any residual solvents contained in the microcapsules and then
   (2) removing the pressurized gas and such residual solvents contained therein.

2. A process according to claim 1 wherein the pressurized gas is selected from $CO_2$ alone and mixtures of $CO_2$ with one of $C_3H_8$, $C_5H_{12}$, and $C_7H_{16}$.

3. A process according to claim 1 wherein the pressurized gas is maintained at a pressure in the range of

about 110 psi to about 250 psi in $CO_2$ pressure alone.

**4.** A process according to claim 2 wherein the pressurized gas is a mixture of $C_3H_8$ and $CO_2$.

**5.** A process according to claim 4 wherein the $C_3H_8$ is present at about 110 psig and the $CO_2$ is present at about 400 psig.

**6.** A process according to claim 1 wherein the pressurized gas is maintained at a constant pressure.

**7.** A process according to claim 1 wherein the polypeptide is a hormonally active polypeptide having LHRH-like activity selected from LHRH and synthetic analogs thereof and is present in the microcapsule composition in an amount of from about 0.01 to about 40.0 weight percent of the polymer used for encapsulation.

**8.** A process according to claim 1 wherein the polymer matrix is a poly(lactic-co-glycolic acid) copolymer (PLGA) having a lactic acid:glycolic acid monomer ratio of 100:0 to about 40:60 and having a molecular weight of from about 20,000 to about 100,000.

**9.** A process according to claim 7 wherein the polypeptide is selected from nafarelin and the pharmaceutically acceptable salts thereof, or N-Ac-D-Nal(2)-D-pCl-Phe-D-Pal(3)-Ser-Tyr-D-Deh-Leu-Deh-Pro-Ala-$NH_2$ and the pharmaceutically acceptable salts thereof.

**10.** A microcapsule product prepared according to the process of Claim 1.